(19) **Europäisches Patentamt / European Patent Office / Office européen des brevets**

(11) **EP 4 686 405 A1**

(12) # EUROPEAN PATENT APPLICATION

(43) Date of publication:
04.02.2026 Bulletin 2026/06

(21) Application number: 25192400.7

(22) Date of filing: 29.07.2025

(51) International Patent Classification (IPC):
A01N 59/16 (2006.01)    A01P 1/00 (2006.01)
A61L 12/08 (2006.01)    A01N 25/30 (2006.01)

(52) Cooperative Patent Classification (CPC):
(C-Sets available)
A01P 1/00; A01N 59/16; G02C 7/04; A61L 12/088;
G02C 2202/24    (Cont.)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH LA MA MD TN

(30) Priority: 01.08.2024 US 202463678143 P

(71) Applicants:
• Largan Medical Co., Ltd.
Taichung City 408 (TW)
• LE Biomedical Corp.
Taoyuan City 330007 (TW)

(72) Inventors:
• CHEN, Wei-Yuan
408 Taichung City (TW)

• SHIH, Yu-Yi
408 Taichung City (TW)
• CHEN, Wei-Chun
408 Taichung City (TW)
• HUANG, Yi Shyang
330007 Taoyuan City (TW)
• NI, Yu Yin
330007 Taoyuan City (TW)
• HONG, Yu Jie
408 Taichung City (TW)
• TENG, Chun-Hung
408 Taichung City (TW)

(74) Representative: Berggren Oy
P.O. Box 16
Fabianinkatu 21
00101 Helsinki (FI)

(54) **CONTACT LENS COMPOSITION AND CONTACT LENS PRODUCT**

(57)    A contact lens composition includes an antibacterial agent and a dispersant. The antibacterial agent is a nanosilver. The dispersant is a polymeric dispersant. When the specific conditions of the percentage of the nanosilver, the percentage of the dispersant and the molecular weight of the dispersant are satisfied, the uniformity of dispersion of the nanosilver can be enhanced.

**EP 4 686 405 A1**

(52) Cooperative Patent Classification (CPC): (Cont.)

C-Sets
**A01N 59/16, A01N 25/30**

## Description

## BACKGROUND

Technical Field

The present disclosure relates to a contact lens composition and a contact lens product. More particularly, the present disclosure relates to a contact lens composition and a contact lens product having an excellent antibacterial effect and an excellent moisturizing.

Description of Related Art

When wearing the contact lens for a long time, the contact lens is prone to rubbing against the eyeball. Further, the reused contact lens is more likely to carry the bacteria thereon, and the user who wears the reused contact lens may have a high probability of redness, swelling, heat, pain and inflammation on the eyeballs thereof. Accordingly, in order to prevent the eyeballs from infecting by the bacteria, the antibacterial agent can be added to the composition of the contact lens, wherein the nanosilver can maintain the antibacterial effect for a long time. However, because of the property of high aggregation of the nanosilver, the nanosilver is hard to evenly distribute in the contact lens, resulting in a reduction of the antibacterial effect. Therefore, to fully realize the antibacterial effect of the nanosilver, a method for dispersing the nanosilver more effectively needs to be developed.

## SUMMARY

According to one aspect of the present disclosure, a contact lens composition includes an antibacterial agent and a dispersant. The antibacterial agent is a nanosilver. The dispersant is a polymeric dispersant. When a percentage of the nanosilver in the contact lens composition is Pag, a percentage of the dispersant in the contact lens composition is Pd, and a molecular weight of the dispersant is MWd, the following conditions are satisfied: $Pag/Pd \leq 0.001$; and $100000 \text{ g/mol} \leq MWd$.

According to the contact lens composition of the foregoing aspect, when the molecular weight of the dispersant is MWd, the following condition can be satisfied: $1000000 \text{ g/mol} \leq MWd$.

According to the contact lens composition of the foregoing aspect, the polymeric dispersant can be a polyvinylpyrrolidone.

According to the contact lens composition of the foregoing aspect, when the percentage of the nanosilver in the contact lens composition is Pag, the percentage of the dispersant in the contact lens composition is Pd, the following condition can be satisfied: $0.0002 \leq Pag/Pd \leq 0.0007$.

According to the contact lens composition of the foregoing aspect, when the percentage of the nanosilver in the contact lens composition is Pag, the following condition can be satisfied: $0.0003\% \leq Pag$.

According to the contact lens composition of the foregoing aspect, when the percentage of the dispersant in the contact lens composition is Pd, the following condition can be satisfied: $0.3\% \leq Pd \leq 3.0\%$.

According to the contact lens composition of the foregoing aspect, when a particle size of the nanosilver is Dag, the following condition can be satisfied: $Dag \leq 100 \text{ nm}$.

According to the contact lens composition of the foregoing aspect, when a particle size of the contact lens composition is Dc, the following condition can be satisfied: $100 \text{ nm} \leq Dc \leq 300 \text{ nm}$.

According to the contact lens composition of the foregoing aspect, when a K value of the dispersant is Kd, the following condition can be satisfied: $50 \leq Kd$.

According to the contact lens composition of the foregoing aspect, when an average transmittance in a wavelength range of 400 nm to 700 nm of the contact lens composition is T4070, the following condition can be satisfied: $90\% \leq T4070$.

According to another aspect of the present disclosure, a contact lens product includes the contact lens composition according to the foregoing aspect and a buffer solution. The contact lens composition is immersed in the buffer solution.

According to further another aspect of the present disclosure, a contact lens composition includes an antibacterial agent, a humectant and a dispersant. The antibacterial agent is a nanosilver. The humectant is a glucan. The dispersant is a polymeric dispersant. When a molecular weight of the dispersant is MWd, the following condition is satisfied: $300000 \text{ g/mol} \leq MWd$.

According to the contact lens composition of the foregoing aspect, when the molecular weight of the dispersant is MWd, the following condition can be satisfied: $500000 \text{ g/mol} \leq MWd$.

According to the contact lens composition of the foregoing aspect, when a percentage of the nanosilver in the contact lens composition is Pag, a percentage of the dispersant in the contact lens composition is Pd, the following condition can be satisfied: Pag/Pd ≤ 0.0008.

According to the contact lens composition of the foregoing aspect, when the percentage of the nanosilver in the contact lens composition is Pag, the following condition can be satisfied: 0.00025% ≤ Pag.

According to the contact lens composition of the foregoing aspect, when the percentage of the dispersant in the contact lens composition is Pd, the following condition can be satisfied: 0.1% ≤ Pd.

According to the contact lens composition of the foregoing aspect, when a particle size of the nanosilver is Dag, the following condition can be satisfied: Dag ≤ 200 nm.

According to the contact lens composition of the foregoing aspect, the polymeric dispersant can be a polyvinylpyrrolidone.

According to the contact lens composition of the foregoing aspect, when a percentage of the humectant in the contact lens composition is Pm, the following condition can be satisfied: 0.5% ≤ Pm ≤ 2.5%.

According to the contact lens composition of the foregoing aspect, when a K value of the dispersant is Kd, the following condition can be satisfied: 30 ≤ Kd.

According to the contact lens composition of the foregoing aspect, when a particle size of the contact lens composition is Dc, the following condition can be satisfied: Dc ≤ 500 nm.

According to the contact lens composition of the foregoing aspect, when an average transmittance in a wavelength range of 400 nm to 700 nm of the contact lens composition is T4070, the following condition can be satisfied: 85% ≤ T4070.

According to the contact lens composition of the foregoing aspect, when a percentage of the nanosilver in the contact lens composition is Pag, a percentage of the dispersant in the contact lens composition is Pd, a percentage of the humectant in the contact lens composition is Pm, a particle size of the nanosilver is Dag, the molecular weight of the dispersant is MWd, a K value of the dispersant is Kd, an average transmittance in a wavelength range of 400 nm to 700 nm of the contact lens composition is T4070, the following conditions can be satisfied: 0.00042% ≤ Pag ≤ 0.00048%; 0.8% ≤ Pd ≤ 1.5%; 1.4% ≤ Pm ≤ 1.6%; 0.0004 ≤ Pag/Pd ≤ 0.0005; 70 nm ≤ Dag ≤ 80 nm; 1200000 g/mol ≤ MWd ≤ 1300000 g/mol; 85 ≤ Kd ≤ 95; and 93% ≤ T4070 ≤ 100%.

According to still another aspect of the present disclosure, a contact lens product includes the contact lens composition according to the foregoing aspect and a buffer solution. The contact lens composition is immersed in the buffer solution.

## DETAILED DESCRIPTION

The present disclosure provides a contact lens composition and a contact lens product. By designing a specific ratio between the nanosilver and the dispersant, an excellent encapsulating effect of the dispersant on the nanosilver can be obtained so as to prevent the nanosilver from aggregating, and thus the uniformity of dispersion of the nanosilver can be enhanced. Further, by using the polymeric dispersant, the nanosilver and other ingredients can be well mixed. Furthermore, the greater the molecular weight of the dispersant, the better the sustained dispersibility thereof. Thus, by adding the dispersant with a specific molecular weight, it is favorable for extending the duration time of dispersibility of the dispersant on the nanosilver. Moreover, by adding the glucan to the contact lens composition of the present disclosure, the moisturizing ability of the contact lens composition can be enhanced, and it is favorable for improving the comfort of the user.

According to one aspect of the present disclosure, a contact lens composition includes an antibacterial agent and a dispersant. The antibacterial agent is a nanosilver. The dispersant is a polymeric dispersant. When a percentage of the nanosilver in the contact lens composition is Pag, a percentage of the dispersant in the contact lens composition is Pd, and a molecular weight of the dispersant is MWd, the following conditions are satisfied: Pag/Pd ≤ 0.001; and 100000 g/mol ≤ MWd. Therefore, by designing a specific ratio between the nanosilver and the dispersant, an excellent encapsulating effect of the dispersant on the nanosilver can be obtained so as to prevent the nanosilver from aggregating, and thus the uniformity of dispersion of the nanosilver can be enhanced. Further, by using the polymeric dispersant, the nanosilver and other ingredients can be well mixed. Furthermore, the greater the molecular weight of the dispersant, the better the sustained dispersibility thereof. Thus, by adding the dispersant with a specific molecular weight, it is favorable for extending the duration time of dispersibility of the dispersant on the nanosilver.

According to another aspect of the present disclosure, a contact lens composition includes an antibacterial agent, a humectant and a dispersant. The antibacterial agent is a nanosilver. The humectant is a glucan. The dispersant is a polymeric dispersant. When a molecular weight of the dispersant is MWd, the following condition is satisfied: 300000 g/mol ≤ MWd. Therefore, by adding the glucan, the moisturizing ability of the contact lens composition can be enhanced, and it is favorable for improving the comfort of the user. Further, by using the polymeric dispersant, the nanosilver and other ingredients can be well mixed. Furthermore, the greater the molecular weight of the dispersant, the better the sustained dispersibility thereof. Thus, by adding the dispersant with a specific molecular weight, it is favorable for extending the duration time of dispersibility of the dispersant on the nanosilver.

When the molecular weight of the dispersant is MWd, the following condition can be satisfied: $1000000 \text{ g/mol} \leq$ MWd. Because the greater the molecular weight of the dispersant, the better the sustained dispersibility thereof will be. Thus, by adding the dispersant with a specific molecular weight, it is favorable for extending the duration time of dispersibility of the dispersant on the nanosilver. Furthermore, the following condition can be satisfied: $500000 \text{ g/mol} \leq \text{MWd}$. Furthermore, the following condition can be satisfied: $800000 \text{ g/mol} \leq \text{MWd}$. Furthermore, the following condition can be satisfied: $1000000 \text{ g/mol} \leq \text{MWd} \leq 1500000 \text{ g/mol}$. Furthermore, the following condition can be satisfied: $1200000 \text{ g/mol} \leq \text{MWd} \leq 1300000 \text{ g/mol}$.

The polymeric dispersant can be a polyvinylpyrrolidone. By the arrangement that the polyvinylpyrrolidone serves as the dispersant, the optimal mixing of the nanosilver and other ingredients can be achieved by the excellent dispersing ability of the polyvinylpyrrolidone.

When the percentage of the nanosilver in the contact lens composition is Pag, and the percentage of the dispersant in the contact lens composition is Pd, the following condition can be satisfied: $0.0002 \leq \text{Pag/Pd} \leq 0.0007$. By designing a specific ratio between the nanosilver and the dispersant, an excellent encapsulating effect of the dispersant on the nanosilver can be obtained so as to prevent the nanosilver from aggregating, and thus the uniformity of dispersion of the nanosilver can be enhanced. Furthermore, the following condition can be satisfied: $\text{Pag/Pd} \leq 0.0008$. Furthermore, the following condition can be satisfied: $0 < \text{Pag/Pd} \leq 0.0008$. Furthermore, the following condition can be satisfied: $0.0003 \leq \text{Pag/Pd} \leq 0.0006$. Furthermore, the following condition can be satisfied: $0.0004 \leq \text{Pag/Pd} \leq 0.0005$.

When the percentage of the nanosilver in the contact lens composition is Pag, the following condition can be satisfied: $0.0003\% \leq \text{Pag}$. By adding the nanosilver with a specific concentration, the contact lens composition has the antibacterial effect, and it is favorable for preventing the contact lens composition from being contaminated by bacteria. Furthermore, the following condition can be satisfied: $0.00025\% \leq \text{Pag}$. Furthermore, the following condition can be satisfied: $0.00033\% \leq \text{Pag} \leq 0.01\%$. Furthermore, the following condition can be satisfied: $0.00035\% \leq \text{Pag} \leq 0.001\%$. Furthermore, the following condition can be satisfied: $0.00038\% \leq \text{Pag} \leq 0.0008\%$. Furthermore, the following condition can be satisfied: $0.0004\% \leq \text{Pag} \leq 0.0005\%$. Furthermore, the following condition can be satisfied: $0.00042\% \leq \text{Pag} \leq 0.00048\%$.

When the percentage of the dispersant in the contact lens composition is Pd, the following condition can be satisfied: $0.3\% \leq \text{Pd} \leq 3.0\%$. By adding the dispersant with a specific concentration, the nanosilver can be dispersed evenly in the contact lens composition, so that it is favorable for enhancing the limpidity of the contact lens composition, and the antibacterial effect can also be dispersed throughout the contact lens composition. Furthermore, the following condition can be satisfied: $0.1\% \leq \text{Pd}$. Furthermore, the following condition can be satisfied: $0.2\% \leq \text{Pd} \leq 5.0\%$. Furthermore, the following condition can be satisfied: $0.5\% \leq \text{Pd} \leq 2.0\%$. Furthermore, the following condition can be satisfied: $0.8\% \leq \text{Pd} \leq 1.5\%$.

When a particle size of the nanosilver is Dag, the following condition can be satisfied: $\text{Dag} \leq 100 \text{ nm}$. By limiting the particle size of the nanosilver, the antibacterial effect of the nanosilver can be enhanced, and it is favorable for prevented the contact lens composition from being contaminated by the bacteria. Furthermore, the following condition can be satisfied: $\text{Dag} \leq 200 \text{ nm}$. Furthermore, the following condition can be satisfied: $\text{Dag} \leq 150 \text{ nm}$. Furthermore, the following condition can be satisfied: $60 \text{ nm} \leq \text{Dag} \leq 90 \text{ nm}$. Furthermore, the following condition can be satisfied: $70 \text{ nm} \leq \text{Dag} \leq 80 \text{ nm}$.

When a particle size of the contact lens composition is Dc, the following condition can be satisfied: $100 \text{ nm} \leq \text{Dc} \leq 300 \text{ nm}$. By limiting the particle size of the contact lens composition, the haze of the contact lens composition can be reduced, and it is favorable for enhancing the limpidity of the contact lens composition. Furthermore, the following condition can be satisfied: $\text{Dc} \leq 500 \text{ nm}$. Furthermore, the following condition can be satisfied: $\text{Dc} \leq 400 \text{ nm}$. Furthermore, the following condition can be satisfied: $150 \text{ nm} \leq \text{Dc} \leq 200 \text{ nm}$. Furthermore, the following condition can be satisfied: $170 \text{ nm} \leq \text{Dc} \leq 190 \text{ nm}$.

When a percentage of the humectant in the contact lens composition is Pm, the following condition can be satisfied: $0.5\% \leq \text{Pm} \leq 2.5\%$. By designing a specific percentage of the humectant in the contact lens composition, the best moisturizing effect of the contact lens composition can be achieved. Furthermore, the following condition can be satisfied: $0\% < \text{Pm} \leq 5.0\%$. Furthermore, the following condition can be satisfied: $0.1\% \leq \text{Pm} \leq 3.0\%$. Furthermore, the following condition can be satisfied: $1.0\% \leq \text{Pm} \leq 2.0\%$. Furthermore, the following condition can be satisfied: $1.3\% \leq \text{Pm} \leq 1.8\%$. Furthermore, the following condition can be satisfied: $1.4\% \leq \text{Pm} \leq 1.6\%$.

When a K value of the dispersant is Kd, the following condition can be satisfied: $50 \leq \text{Kd}$. By satisfying the K value of the dispersant, the dispersing ability of the dispersant can be enhanced, and it is favorable for further enhancing the uniformity of dispersion of the nanosilver. Furthermore, the following condition can be satisfied: $30 \leq \text{Kd}$. Furthermore, the following condition can be satisfied: $20 \leq \text{Kd}$. Furthermore, the following condition can be satisfied: $70 \leq \text{Kd}$. Furthermore, the following condition can be satisfied: $80 \leq \text{Kd} \leq 100$. Furthermore, the following condition can be satisfied: $85 \leq \text{Kd} \leq 95$.

When an average transmittance in a wavelength range of 400 nm to 700 nm of the contact lens composition is T4070, the following condition can be satisfied: $90\% \leq \text{T4070}$. By limiting the average transmittance of the contact lens composition in the wavelength range of 400 nm to 700 nm, the penetration of the visible light can be enhanced, and it is favorable for avoiding the obstruction of the sightline of the user. Furthermore, the following condition can be satisfied: 85% $\leq$ T4070. Furthermore, the following condition can be satisfied: 88% $\leq$ T4070. Furthermore, the following condition can be satisfied: 92% $\leq$ T4070. Furthermore, the following condition can be satisfied: 93% $\leq$ T4070 $\leq$ 100%.

The contact lens composition of the present disclosure can be a formulated solution of the contact lens before curing or the cured contact lens. The aforementioned curing can be light curing or heat curing.

The component of the contact lens composition of the present disclosure includes the internal component and the surface component. The internal component of the contact lens composition means that the ingredients of the contact lens composition are served as the materials. The surface component of the contact lens composition means that the ingredients of the contact lens composition are bound or attached to the surface of the contact lens composition by coating, soaking or other methods. The component of the contact lens composition can include at least one, at least two, at least three or at least four antibacterial agents. The component of the contact lens composition can include at least one, at least two, at least three or at least four dispersants. The component of the contact lens composition can include at least one, at least two, at least three or at least four humectants. The component of the contact lens composition can include at least one, at least two, at least three or at least four beneficial agents. The component of the contact lens composition can include at least one, at least two, at least three or at least four antioxidants. The component of the contact lens composition can include at least one, at least two, at least three or at least four myopia controlling agents. The component of the contact lens composition can include at least one, at least two, at least three or at least four surfactants. The component of the contact lens composition can include at least one, at least two, at least three or at least four cooling agents. The component of the contact lens composition can include at least one, at least two, at least three or at least four eye-protecting agents. The component of the contact lens composition can include at least one, at least two, at least three or at least four buffering agents. The component of the contact lens composition can include at least one, at least two, at least three or at least four chelating agents. The component of the contact lens composition can include at least one, at least two, at least three or at least four monomers. The component of the contact lens composition can include at least one, at least two, at least three or at least four initiators. The component of the contact lens composition can include at least one, at least two, at least three or at least four cross-linking agents. The component of the contact lens composition can include at least one, at least two, at least three or at least four diluents. The component of the contact lens composition can include at least one, at least two, at least three or at least four UV absorbers or blue light absorbers. The component of the contact lens composition can include at least one, at least two, at least three or at least four pigments.

The percentage of each component in the contact lens composition of the present disclosure is calculated with the weight percentage as the unit. When the contact lens composition is the contact lens, the contact lens composition should be completely dehydrated so as to calculate the percentage of each component.

The antibacterial agent of the present disclosure can include nanosilver, silver ion ($Ag^+$), benzoic acid, sodium nitrite, calcium propionate, boric acid, sodium borate, methylparaben, DMDM hydantoin (DMDMH), polycapramide (polyhexamethyleneguanidine polisept, PHMB), etc., and the salts thereof. The nanosilver refers to the silver atom having the particle size smaller than 1000 nm.

The particle size of the present disclosure is the Z-average results obtained by the dynamic light scattering particle size analyzer, and the formula thereof is $Dz = \Sigma Si/\Sigma(Si/Di)$, wherein Dz is the calculated result of the particle size (can be substituted into the particle size Dag of the nanosilver and the particle size Dc of the contact lens composition of the present disclosure), Si is the scattering intensity of a single particle, and Di is the diameter of a single particle.

The dispersant of the present disclosure can include cationic dispersant, anionic dispersant, nonionic dispersant, amphoteric dispersant, charge-neutral dispersant, polymeric dispersant or free radical dispersant. The cationic dispersant can include ammonium salt, quaternary ammonium salt, pyridinium salt, etc. The anionic dispersant can include sodium oleate ($C_{17}H_{33}COONa$), carboxylates, organosulfates ($R-O-SO_3Na$), sulfonates ($R-SO_3Na$), etc. The nonionic dispersant can include ethylene glycol or polyol. The amphoteric dispersant can include phosphate ester salt. The charge-neutral dispersant can include oleylamino oleate ($C_{18}H_{35}NH_3OOCC_{17}H_{33}$). The polymeric dispersant means the polymer dispersant and can include polyvinylpyrrolidone (PVP), Hypermer B246, polycaprolactone polyol-polyethyleneimine block copolymer, acrylate polymer, polyurethane, polyester, etc.

The molecular weight of the dispersant of the present disclosure means the weight-average molecular weight, and the formula of the weight-average molecular weight is $Mw = (\Sigma NiMi^2)/(\Sigma NiMi)$, wherein Mw is the weight-average molecular weight (can be substituted into the molecular weight MWd of the dispersant of the present disclosure), Ni is the molecule number of the corresponding molecular weight, and Mi is the molecular weight of a single molecule.

The K value of the dispersant of the present disclosure is an indicator of the average molecular size based on the relative viscosity and the solubility. The K value is calculated based on the Fikentscher formula, and the Fikentscher formula is $K = \{[300Clog\eta+(C+1.5log\eta)^2]^{1/2}+1.5log\eta-C\}/(0.15C+0.003C^2)$, wherein $\eta$ is the relative viscosity, C is the number of grams of the solute dissolved in 100 ml of the solution.

The humectant of the present disclosure can include glucan, PEG 40 Castor Oil, PEG 400, glycerol, allantoin, hyaluronic acid, ceramide, cholesterol, sea buckthorn oil, polyglutamic acid ($\gamma$-PGA), trehalose, alginic acid, hydroxypropyl methylcellulose (HPMC), 2-methacryloyloxyethyl phosphorylcholine (MPC), etc., and the salts thereof.

The beneficial agent of the present disclosure can include antibotic, bacteriostatic agent, antiviral agent, antifungal drugs, antiallergic agent, apomorphine, bromocriptine, dopamine receptor agonists, levodopa, quinpirole, steroid, nonsteroidal anti-inflammatory drugs (NSAIDs), surfactants, miotics, enzyme inhibitor, anaesthetic, vasoconstrictor, nutrient, etc.

The antioxidant of the present disclosure can include vitamin A, vitamin C, vitamin D, vitamin E, uric acid, carotenoid, carotene, lutein, flavonoids, resveratrol, selenomethionine, oxidized or reduced coenzyme Q10, glutathione, thioctic acid, dibutylated hydroxytoluene (BHT), butylated hydroxyanisole (BHA), gallic acid propyl ester (PG), tert-butylhydroquinone (TBHQ), etc., and the salts thereof.

The myopia controlling agent of the present disclosure has the effects of controlling, slowing down, delaying or preventing the worsening of myopia. The myopia controlling agent can include cycloplegics, mydriatics, selective/non-selective muscarinic receptor antagonists, etc., such as atropine ((3-endo)-8-methyl-8-azabicyclo[3.2.1]oct-3-yltropate), atropine sulphate, cyclopentolate (2-(dimethylamino)ethyl(1-hydroxycyclopentyl)(phenyl)acetate), cyclopentolate HCl, eucatropine (1,2,2,6-tetramethyl-4-piperidinylhydroxy(phenyl)acetate), homatropine ((3-endo)-8-methyl-8-azabicyclo [3.2.1]oct-3-ylhydroxy(phenyl)acetate), nuvenzepine, phenylephrine HCl, pirenzepine, raceanisodamine, rispenzepine, scopolamine ((1R,2R,4S,5S,7s)-9-methyl-3-oxa-9-azatricyclo[3.3.1.02,4]non-7-yl(2S)-3-hydro xy-2-phenylpropanoate), scopolamine HBr, telenzepine and tropicamide (N-ethyl-3-hydroxy-2-phenyl-N-(4-pyridinylmethyl)propanamide), etc., and the salts thereof.

The surfactant of the present disclosure can include poloxamer 407 (KP407), sodium dodecyl sulfate (SDS), cetyltrimethylammonium bromide (CTAB), trimethylstearylammonium chioride (STAC), potassium stearate, acacia gum, sodium alginate, etc., and the salts thereof.

The cooling agent of the present disclosure has a cooling effect on the affected area. The cooling agent can include menthol, 2-isopropyl-N,2,3-trimethylbutyramide (WS-23), N-ethyl-p-menthane-3-carboxamide (WS-3), ethyl 3-(p-menthane-3-carboxamido)acetate (WS-5), (1R,2S,5R)-N-(4-methoxyphenyl)-p-menthanecarboxamide (WS-12), N-ethyl-2,2-diisopropylbutanamide (WS-27), N-(1,1-dimethyl-2-hydroxyethyl)-2,2-diethylbutanamide (WS-116), etc., and the salts thereof.

The eye-protecting agent of the present disclosure can include vitamin A, vitamin B, lutein, omega-3 fatty acid, anthocyanins, astaxanthin, carotene, bilberry, lecithin, or the combination thereof.

The buffering agent of the present disclosure can include buffering salts, such as citrate, acetate, phosphate, borate, sulfate, carbonate, nitrate, chloride, or the combination thereof. The cationic of the buffering salts can include sodium ion, potassium ion, magnesium ion and calcium ion. The buffering agent can include N-[tris(hydroxymethyl) methyl]-3-aminopropanesulfonic acid (TAPS), N,N-bis(2-hydroxyethyl)glycine (Bicine), tris(hydroxymethyl)aminomethane (Tris), N-tris(hydroxymethyl) methylglycine (Tricine), 4-(2-hydroxyethyl)piperazine-1-ethanesulfonic acid hemisodium salt (HEPES), N-tris(hydroxymethyl)methyl-2-aminoethanesulfonic acid (TES), 3-(N-morpholino)propanesulfonic acid (MOPS), 1,4-piperazinediethanesulfonic acid (PIPES), cacodylate, 2-morpholinoethanesulphonic acid (MES), etc., and the salts thereof.

The chelating agent of the present disclosure can include ethylenediamine (en), 2,2'-bipyridine (bipy), 1,10-phenanthroline (phen), oxalic acid, ethylenediaminetetraacetic acid (EDTA), 1,2-bis(dimethylarsino)benzene (diars), etc., and the salts thereof.

The monomer of the present disclosure means the ingredient of the contact lens composition which can provide the structural supportability, and the monomer can be the hydrogel monomer or the silicone hydrogel monomer. The hydrogel monomer can include 2-hydroxyethyl methacrylate (HEMA), methacrylic acid (MAA), glycerol monomethacrylate (GMA), N-vinyl-2-pyrrolidinone (NVP), methyl methacrylate (MMA), N,N-dimethyl acrylamide (DMAA), etc. The silicone hydrogel monomer can include 2-hydroxyethyl methacrylate, glycerol monomethacrylate, methacrylic acid, 3-methacryloyloxypropyltris(trimethylsilyloxy) silane, N-vinyl-2-pyrrolidinone, N,N-dimethyl acrylamide, 3-(3-methacryloxy-2-hydroxypropoxy)propylbis(trimethylsiloxy)methylsilane or (3-acryloxy-2-hydroxypropoxypropyl) terminated polydimethylsiloxane.

The initiator of the present disclosure can be 2-hydroxy-2-methyl-propiophenone or phenylbis(2,4,6-trimethylbenzoyl)phosphine oxide.

The cross-linking agent of the present disclosure can be ethylene glycol dimethacrylate (EGDMA) or 1,1,1-trimethylol propane trimethacrylate (TMPTA).

The diluent of the present disclosure can be polyethylene glycol 300, polyethylene glycol 600, polyethylene glycol 800, polyethylene glycol 1000, polyethylene glycol 2000, polyethylene glycol 4000, 1,4-butanediol, ethanol, isopropyl alcohol, glycerol or 1-hexanol.

The ultraviolet (UV) absorber of the present disclosure can be 2,4-dihydroxybenzophenone (BP1), 2-[3-(2H-benzotriazol-2-yl)-4-hydroxyphenyl]ethyl methacrylate, 4-methacryloxy-2-hydroxybenzophenone, 2-phenylethyl acrylate, 2-phenylethyl methacrylate, 2-(2'-hydroxy-5'-methacryloxyethylphenyl)-2H-benzotriazole or 2-(4-benzoyl-3-hydroxyphenoxy) ethyl acrylate.

The blue light absorber of the present disclosure can be 4-(phenyldiazenyl) phenyl methacrylate.

The pigment of the present disclosure can include anthocyanidin, Beta-carotene, curcumin, luciferin, lutein, lycopene, phycobillin, phycoerythrim, phycocyanin, vitamin B2, zeaxanthin, photochromic dyes, thermochromic dyes, etc., and the derivatives thereof.

The material of the contact lens composition of the present disclosure can be divided into the hydrogel and the silicone hydrogel. The hydrogel can be the contact lens material classified as Group I by U.S. FDA, i.e., nonionic polymers having a low moisture content (less than 50 wt%), such as Helfilcon A&B, Hioxifilcon B, Mafilcon, Polymacon, Tefilcon, Tetrafilcon A, etc. Furthermore, the hydrogel can be the contact lens material classified as Group II by U.S. FDA, i.e., nonionic polymers having a high moisture content (greater than 50 wt%), such as Acofilcon A, Alfafilcon A, Hilafilcon B, Hioxifilcon A, Hioxifilcon B, Hioxifilcon D, Nelfilcon A, Nesofilcon A, Omafilcon A, Samfilcon A, etc. Furthermore, the hydrogel can be the contact lens material classified as Group III by U.S. FDA, i.e., ionic polymers having a low moisture content (less than 50 wt%), such as Deltafilcon A, etc. Furthermore, the hydrogel can be the contact lens material classified as Group IV by U.S. FDA, i.e., ionic polymers having a high moisture content (greater than 50 wt%), such as Etafilcon A, Focofilcon A, Methafilcon A, Methafilcon B, Ocufilcon A, Ocufilcon B, Ocufilcon C, Ocufilcon D, Ocufilcon E, Phemfilcon A, Vifilcon A, etc. The silicone hydrogel can be the contact lens material classified as Group V by U.S. FDA, such as Balafilcon A, Comfilcon A, Efrofilcon A, Enfilcon A, Galyfilcon A, Lotrafilcon A, Lotrafilcon B, Narafilcon A, Narafilcon B, Senofilcon A, Delefilcon A, Somofilcon A, etc.

The average transmittance of the contact lens composition of the present disclosure is the average of the transmittances of the integer wavelengths at an interval of 1 nm. The average transmittance in a wavelength range of 400 nm to 700 nm is the average of the transmittances of the wavelengths at an interval of 1 nm from 400 nm to 700 nm, that is, take the average of the transmittance at 400 nm, 401 nm, 402 nm, 403 nm, and so on up to 700 nm. The average transmittance in a wavelength range of 450 nm to 630 nm is the average of the transmittances of the wavelengths at an interval of 1 nm from 450 nm to 630 nm, that is, take the average of the transmittance at 450 nm, 451 nm, 452 nm, 453 nm, and so on up to 630 nm. When the contact lens composition is the formulated solution of the contact lens, 1 $\mu$l to 2 $\mu$l or 1 ml to 2 ml is taken for analysis according to the instrument requirements. When the contact lens composition is the contact lens, the center of mass thereof is taken for analysis when the contact lens is laid flat.

Each of the aforementioned features of the contact lens composition of the present disclosure can be utilized in numerous combinations, so as to achieve the corresponding functionality.

According to further another embodiment of the present disclosure, a contact lens product includes the aforementioned contact lens composition and a buffer solution. The contact lens composition is immersed in the buffer solution.

The structure of the contact lens of the present disclosure can be a single-layered contact lens, and also can be a contact lens with at least two layers, wherein the color contact lens can be composed of two layers, such as the lens body layer and the color layer; the color contact lens can be composed of three layers, such as the lens body layer, the color layer and the anti-shedding protective layer; the color contact lens can be composed of four layers, such as the lens body layer, the first color layer, the second color layer and the anti-shedding protective layer. The color contact lens can be composed of five layers, such as the lens body layer, the first color layer, the second color layer, the third color layer and the anti-shedding protective layer. The color contact lens can be composed of another type of the five layers, such as the lens body layer, the first color layer, the spacing layer, the second color layer and the anti-shedding protective layer.

The stability structure of the contact lens of the present disclosure can include a rotating stabilizing design, such as a lower weighted design by thickening on the lower part of the contact lens, a balanced design by thickening on both sides thereof, an asymmetrical balanced design thereof, a stable design by thinning the upper and lower portions thereof, etc.

The function of the contact lens of the present disclosure can be myopia correction, hyperopia correction, presbyopia correction, astigmatism correction, myopia control, corneal reshaping, etc.

The contact lens product of the present disclosure can be composed of a contact lens composition, a buffer solution and a package. The contact lens composition is immersed in the buffer solution. The contact lens composition and the buffer solution can include an antibacterial agent, a dispersant, a humectant, a beneficial agent, an antioxidant, a myopia controlling agent, a surfactant, a cooling agent, an eye-protecting agent, a buffering agent, a chelating agent, a diluent, an UV absorber, a blue light absorber or a pigment. The package can be made by assembling a plastic container and an aluminum foil cover, wherein the plastic container can be made of polypropylene (PP), polystyrene (PS), polyethylene terephthalate (PET) or other plastic materials. Further, it also can be made of biodegradable plastic materials, such as polylactic acid (PLA), polyhydroxyalkanoates (PHA) or other degradable plastic materials. Furthermore, the biodegradable plastic material can also be mixed with biodegradable plasticizers to achieve effects of modification. The aluminum foil cover of the present disclosure can be a composite aluminum foil material with a plastic coating.

The antibacterial activity of the present disclosure means the ability to resist bacterial growth or kill bacteria. The test of the antibacterial activity is designed with reference to ASTM E 2149-20, and the steps are shown as follows: (1) a plastic sheet with the size of 5 cm × 5 cm and the weight of 1.05 g approximately is made based on the percentage of the component of the contact lens composition; (2) the plastic sheet is sterilized with the UV light; (3) the plastic sheet is soaked in 50 ml sterile water for 24 hours; (4) the plastic sheet is taken out, and after making sure that the plastic sheet is not dripping, the plastic sheet is cut it into pieces and put into a conical flask; (5) 50 ml of the bacterial liquid is added to the conical flask and co-cultured with the plastic sheet being cut for 24 hours, wherein the bacterium of the bacterial liquid is *Escherichia coli* (ATCC 25922); and (6) the bacterial solution in the conical flask is diluted appropriately and spread on a culture medium suitable for bacterial growth, and the culture medium is cultured at 35°C for 24 hours. The formula of the antibacterial activity = (b-a)/b×100%, CFU, wherein CFU = colony forming unit, b is the number of bacteria cultured in the bacterial liquid alone, and a is the number of bacteria obtained from co-culturing the bacterial liquid and the plastic sheet. When the antibacterial activity is >50%, it represents that the component has an antibacterial effect, and when the percentage of the antibacterial activity is higher, the antibacterial effect is better. Further, the testing conditions of the test, which are not shown in the aforementioned description, are in accordance with ASTM E 2149-20.

The measurement environment of the present disclosure is 25°C and 50% humidity unless otherwise specified.

According to the above descriptions, the specific embodiments are given below so as to describe the present disclosure in detail.

<Comparative example 1>

The contact lens composition of Comparative example 1 is the formulated solution of the contact lens, and the formulated solution of the contact lens forms a contact lens after curing.

The contact lens composition of Comparative example 1 includes a dispersant but does not include an antibacterial agent and a humectant, wherein the dispersant of Comparative example 1 is polyvinylpyrrolidone, and the components and the percentage of each of the components of Comparative example 1 are shown in Table 1.

| Table 1 | | | |
|---|---|---|---|
| Pag (%) | - | Precipitation time of nanosilver (Day) | - |
| Pd (%) | 1 | T4070 (%) | - |
| Pm (%) | - | Nanosilver evenly dispersed | × |
| Pag/Pd | - | Antibacterial activity (%) | × |
| Dag (nm) | - | | |
| Dc (nm) | - | | |
| MWd (g/mol) | 1280000 | | |
| Kd | 90 | | |

In Table 1, Pag is a percentage of the nanosilver in the contact lens composition, Pd is a percentage of the dispersant in the contact lens composition, Pm is a percentage of the humectant in the contact lens composition, Dag is a particle size of the nanosilver, Dc is a particle size of the contact lens composition, MWd is a molecular weight of the dispersant, Kd is a K value of the dispersan, and T4070 is an average transmittance in a wavelength range of 400 nm to 700 nm of the contact lens composition. Further, the symbol "-" in Table 1 means that there is no test data.

As shown in Table 1, the contact lens composition of Comparative example 1 after curing to form the contact lens is without the antibacterial activity.

If the definitions of parameters shown in tables of the following comparative example and examples are the same as those shown in Table 1, those will not be described again.

<Comparative example 2>

The contact lens composition of Comparative example 2 is the formulated solution of the contact lens, and the formulated solution of the contact lens forms a contact lens after curing.

The contact lens composition of Comparative example 2 includes an antibacterial agent but does not include a dispersant and a humectant, wherein the antibacterial agent of Comparative example 2 is the nanosilver, and the components and the percentage of each of the components Comparative example 2 are shown in Table 2.

| Table 2 | | | |
|---|---|---|---|
| Pag (%) | - | Precipitation time of nanosilver (Day) | - |
| Pd (%) | - | T4070 (%) | - |
| Pm (%) | 0.00034 | Nanosilver evenly dispersed | × |
| Pag/Pd | - | Antibacterial activity (%) | ○ |
| Dag (nm) | 76.13 | | |
| Dc (nm) | - | | |
| MWd (g/mol) | - | | |
| Kd | - | | |

As shown in Table 2, the contact lens composition of Comparative example 2 after curing to form the contact lens has the antibacterial activity, but the nanosilver thereof is not evenly dispersed.

<Example 1>

The contact lens composition of Example 1 is the formulated solution of the contact lens, and the formulated solution of the contact lens forms a contact lens after curing.
The contact lens composition of Example 1 includes an antibacterial agent and a dispersant but does not include a humectant, wherein the antibacterial agent is the nanosilver, the dispersant is the polyvinylpyrrolidone, and the components and the percentage of each of the components of Example 1 are shown in Table 3.

| Table 3 | | | |
|---|---|---|---|
| Pag (%) | 0.00034 | Precipitation time of nanosilver (Day) | 21 |
| Pd (%) | 1 | T4070 (%) | - |
| Pm (%) | - | Nanosilver evenly dispersed | ○ |
| Pag/Pd | 0.00034 | Antibacterial activity (%) | - |
| Dag (nm) | 76.13 | | |
| Dc (nm) | 768.1 | | |
| MWd (g/mol) | 34000 | | |
| Kd | 25 | | |

As shown in Table 3, the nanosilver of the contact lens composition of Example 1 begins to precipitate after standing for 21 days, and the nanosilver of the contact lens composition after curing to form the contact lens is evenly dispersed.

<Example 2>

The contact lens composition of Example 2 is the formulated solution of the contact lens, and the formulated solution of the contact lens forms a contact lens after curing.
The contact lens composition of Example 2 includes an antibacterial agent and a dispersant but does not include a humectant, wherein the antibacterial agent is the nanosilver, the dispersant is the polyvinylpyrrolidone, and the components and the percentage of each of the components of Example 2 are shown in Table 4.

| Table 4 | | | |
|---|---|---|---|
| Pag (%) | 0.00034 | Precipitation time of nanosilver (Day) | 28 |
| Pd (%) | 1 | T4070 (%) | - |
| Pm (%) | - | Nanosilver evenly dispersed | ○ |

(continued)

| Table 4 | | | |
|---|---|---|---|
| Pag/Pd | 0.00034 | Antibacterial activity (%) | - |
| Dag (nm) | 76.13 | | |
| Dc (nm) | 263 | | |
| MWd (g/mol) | 400000 | | |
| Kd | 60 | | |

As shown in Table 4, the nanosilver of the contact lens composition of Example 2 begins to precipitate after standing for 28 days, and the nanosilver of the contact lens composition after curing to form the contact lens is evenly dispersed.

<Example 3>

The contact lens composition of Example 3 is the formulated solution of the contact lens, and the formulated solution of the contact lens forms a contact lens after curing.

The contact lens composition of Example 3 includes an antibacterial agent, a dispersant and a humectant, wherein the antibacterial agent is the nanosilver, the dispersant is the polyvinylpyrrolidone, the humectant is the glucan, and the components and the percentage of each of the components of Example 3 are shown in Table 5.

| Table 5 | | | |
|---|---|---|---|
| Pag (%) | 0.000272 | Precipitation time of nanosilver (Day) | 14 |
| Pd (%) | 0.4 | T4070 (%) | 92.41 |
| Pm (%) | 1.5 | Nanosilver evenly dispersed | ○ |
| Pag/Pd | 0.00068 | Antibacterial activity (%) | - |
| Dag (nm) | 76.13 | | |
| Dc (nm) | 215.7 | | |
| MWd (g/mol) | 1280000 | | |
| Kd | 90 | | |

As shown in Table 5, the nanosilver of the contact lens composition of Example 3 begins to precipitate after standing for 14 days, and the nanosilver of the contact lens composition after curing to form the contact lens is evenly dispersed.

<Example 4>

The contact lens composition of Example 4 is the formulated solution of the contact lens, and the formulated solution of the contact lens forms a contact lens after curing.

The contact lens composition Example 4 includes an antibacterial agent, a dispersant and a humectant, wherein the antibacterial agent is the nanosilver, the dispersant is the polyvinylpyrrolidone, the humectant is the glucan, and the components and the percentage of each of the components of Example 4 are shown in Table 6A.

| Table 6A | | | |
|---|---|---|---|
| Pag (%) | 0.0003 | Precipitation time of nanosilver (Day) | 14 |
| Pd (%) | 0.4 | T4070 (%) | 92.62 |
| Pm (%) | 1.5 | Nanosilver evenly dispersed | ○ |
| Pag/Pd | 0.00075 | Antibacterial activity (%) | 99.69 |

(continued)

| Table 6A | |
|---|---|
| Dag (nm) | 76.13 |
| Dc (nm) | 210.2 |
| MWd (g/mol) | 1280000 |
| Kd | 90 |

As shown in Table 6A, the nanosilver of the contact lens composition of Example 4 begins to precipitate after standing for 14 days, and the nanosilver of the contact lens composition after curing to form the contact lens is evenly dispersed.

Further, the results of the antibacterial activity test of the contact lens composition of Example 4 according to the steps described in the aforementioned paragraph are shown in Table 6B.

| Table 6B | |
|---|---|
| Test strain | *Escherichia coli* |
| Deposit number | ATCC 25922 |
| Initial bacterial number | $2.5 \times 10^5$ CFU/ ml |
| Number of bacteria cultured in bacterial liquid alone | $1.8 \times 10^6$ CFU/ ml |
| Number of bacteria obtained from co-culturing bacterial liquid and plastic sheet | $5.6 \times 10^3$ CFU/ ml |
| Antibacterial activity | 99.69% |

<Example 5>

The contact lens composition of Example 5 is the formulated solution of the contact lens, and the formulated solution of the contact lens forms a contact lens after curing.

The contact lens composition of Example 5 includes an antibacterial agent and a dispersant but does not include a humectant, wherein the antibacterial agent is the nanosilver, the dispersant is the polyvinylpyrrolidone, and the components and the percentage of each of the components of Example 5 are shown in Table 7.

| Table 7 | | | |
|---|---|---|---|
| Pag (%) | 0.00034 | Precipitation time of nanosilver (Day) | >28 |
| Pd (%) | 1 | T4070 (%) | - |
| Pm (%) | - | Nanosilver evenly dispersed | ○ |
| Pag/Pd | 0.00034 | Antibacterial activity (%) | - |
| Dag (nm) | 76.13 | | |
| Dc (nm) | - | | |
| MWd (g/mol) | 1280000 | | |
| Kd | 90 | | |

As shown in Table 7, the nanosilver of the contact lens composition of Example 5 begins to precipitate after standing for longer than 28 days, and the nanosilver of the contact lens composition after curing to form the contact lens is evenly dispersed.

<Example 6>

The contact lens composition of Example 6 is the formulated solution of the contact lens, and the formulated solution of the contact lens forms a contact lens after curing.

The contact lens composition of Example 6 includes an antibacterial agent and a dispersant but does not include a humectant, wherein the antibacterial agent is the nanosilver, the dispersant is the polyvinylpyrrolidone, and the components and the percentage of each of the components of Example 6 are shown in Table 8.

| Table 8 | | | |
|---|---|---|---|
| Pag (%) | 0.00045 | Precipitation time of nanosilver (Day) | >28 |
| Pd (%) | 1 | T4070 (%) | - |
| Pm (%) | - | Nanosilver evenly dispersed | ○ |
| Pag/Pd | 0.00045 | Antibacterial activity (%) | - |
| Dag (nm) | 76.13 | | |
| Dc (nm) | - | | |
| MWd (g/mol) | 1280000 | | |
| Kd | 90 | | |

As shown in Table 8, the nanosilver of the contact lens composition of Example 6 begins to precipitate after standing for longer than 28 days, and the nanosilver of the contact lens composition after curing to form the contact lens is evenly dispersed.

<Example 7>

The contact lens composition of Example 7 is the formulated solution of the contact lens, and the formulated solution of the contact lens forms a contact lens after curing.

The contact lens composition of Example 7 includes an antibacterial agent, a dispersant and a humectant, wherein the antibacterial agent is the nanosilver, the dispersant is the polyvinylpyrrolidone, the humectant is the glucan, and the components and the percentage of each of the components of Example 7 are shown in Table 9.

| Table 9 | | | |
|---|---|---|---|
| Pag (%) | 0.00034 | Precipitation time of nanosilver (Day) | 18 |
| Pd (%) | 1 | T4070 (%) | 93.07 |
| Pm (%) | 1.5 | Nanosilver evenly dispersed | ○ |
| Pag/Pd | 0.00034 | Antibacterial activity (%) | - |
| Dag (nm) | 76.13 | | |
| Dc (nm) | 181.6 | | |
| MWd (g/mol) | 1280000 | | |
| Kd | 90 | | |

As shown in Table 9, the nanosilver of the contact lens composition of Example 7 begins to precipitate after standing for 18 days, and the nanosilver of the contact lens composition after curing to form the contact lens is evenly dispersed.

<Example 8>

The contact lens composition of Example 8 is the formulated solution of the contact lens, and the formulated solution of the contact lens forms a contact lens after curing.

The contact lens composition of Example 8 includes an antibacterial agent, a dispersant and a humectant, wherein the antibacterial agent is the nanosilver, the dispersant is the polyvinylpyrrolidone, the humectant is the glucan, and the components and the percentage of each of the components of Example 8 are shown in Table 10.

| Table 10 | | | |
|---|---|---|---|
| Pag (%) | 0.00045 | Precipitation time of nanosilver (Day) | 18 |
| Pd (%) | 1 | T4070 (%) | 93.18 |
| Pm (%) | 1.5 | Nanosilver evenly dispersed | ○ |
| Pag/Pd | 0.00045 | Antibacterial activity (%) | - |
| Dag (nm) | 76.13 | | |
| Dc (nm) | 186.9 | | |
| MWd (g/mol) | 1280000 | | |
| Kd | 90 | | |

As shown in Table 10, the nanosilver of the contact lens composition of Example 8 begins to precipitate after standing for 18 days, and the nanosilver of the contact lens composition after curing to form the contact lens is evenly dispersed.

<Example 9>

The contact lens composition of Example 9 is the formulated solution of the contact lens, and the formulated solution of the contact lens forms a contact lens after curing.

The contact lens composition of Example 9 includes an antibacterial agent and a dispersant but does not include a humectant, wherein the antibacterial agent is the nanosilver, the dispersant is the polyvinylpyrrolidone, and the components and the percentage of each of the components of Example 9 are shown in Table 11.

| Table 11 | | | |
|---|---|---|---|
| Pag (%) | 0.00034 | Precipitation time of nanosilver (Day) | >28 |
| Pd (%) | 2 | T4070 (%) | - |
| Pm (%) | - | Nanosilver evenly dispersed | ○ |
| Pag/Pd | 0.00017 | Antibacterial activity (%) | - |
| Dag (nm) | 76.13 | | |
| Dc (nm) | - | | |
| MWd (g/mol) | 1280000 | | |
| Kd | 90 | | |

As shown in Table 11, the nanosilver of the contact lens composition of Example 9 begins to precipitate after standing for longer than 28 days, and the nanosilver of the contact lens composition after curing to form the contact lens is evenly dispersed.

<Example 10>

The contact lens composition of Example 10 is the formulated solution of the contact lens, and the formulated solution of the contact lens forms a contact lens after curing.

The contact lens composition of Example 10 includes an antibacterial agent and a dispersant but does not include a humectant, wherein the antibacterial agent is the nanosilver, the dispersant is the polyvinylpyrrolidone, and the components and the percentage of each of the components of Example 10 are shown in Table 12.

| Table 12 | | | |
|---|---|---|---|
| Pag (%) | 0.00045 | Precipitation time of nanosilver (Day) | >28 |
| Pd (%) | 3 | T4070 (%) | - |
| Pm (%) | - | Nanosilver evenly dispersed | ○ |

(continued)

| Table 12 | | | |
|---|---|---|---|
| Pag/Pd | 0.00015 | Antibacterial activity (%) | - |
| Dag (nm) | 76.13 | | |
| Dc (nm) | - | | |
| MWd (g/mol) | 1280000 | | |
| Kd | 90 | | |

As shown in Table 12, the nanosilver of the contact lens composition of Example 10 begins to precipitate after standing for longer than 28 days, and the nanosilver of the contact lens composition after curing to form the contact lens is evenly dispersed.

**Claims**

1. A contact lens composition, **characterized in** comprising:

   an antibacterial agent being a nanosilver; and
   a dispersant being a polymeric dispersant;
   wherein a percentage of the nanosilver in the contact lens composition is Pag, a percentage of the dispersant in the contact lens composition is Pd, a molecular weight of the dispersant is MWd, and the following conditions are satisfied:

$$Pag/Pd \leq 0.001;$$

   and

$$100000 \text{ g/mol} \leq MWd.$$

2. The contact lens composition of claim 1, wherein the molecular weight of the dispersant is MWd, and the following condition is satisfied:
   $1000000 \text{ g/mol} \leq MWd$.

3. The contact lens composition of claims 1 or 2, wherein the polymeric dispersant is a polyvinylpyrrolidone.

4. The contact lens composition of any one of claims 1 to 3, wherein the percentage of the nanosilver in the contact lens composition is Pag, the percentage of the dispersant in the contact lens composition is Pd, and the following condition is satisfied:

$$0.0002 \leq Pag/Pd \leq 0.0007.$$

5. The contact lens composition of any one of claims 1 to 4, wherein the percentage of the nanosilver in the contact lens composition is Pag, and the following condition is satisfied:

$$0.0003\% \leq Pag.$$

6. The contact lens composition of any one of claims 1 to 5, wherein the percentage of the dispersant in the contact lens composition is Pd, and the following condition is satisfied:

$$0.3\% \leq Pd \leq 3.0\%.$$

7. The contact lens composition of any one of claims 1 to 6, wherein a particle size of the nanosilver is Dag, and the following condition is satisfied:

$$Dag \leq 100 \text{ nm}.$$

8. The contact lens composition of any one of claims 1 to 7, wherein a particle size of the contact lens composition is Dc, and the following condition is satisfied:

$$100 \text{ nm} \leq Dc \leq 300 \text{ nm}.$$

9. The contact lens composition of any one of claims 1 to 8, wherein a K value of the dispersant is Kd, and the following condition is satisfied:

$$50 \leq Kd.$$

10. The contact lens composition of any one of claims 1 to 9, wherein an average transmittance in a wavelength range of 400 nm to 700 nm of the contact lens composition is T4070, and the following condition is satisfied:

$$90\% \leq T4070.$$

11. A contact lens product, **characterized in** comprising:

the contact lens composition of any one of claims 1 to 10; and
a buffer solution, wherein the contact lens composition is immersed in the buffer solution.

12. A contact lens composition, **characterized in** comprising:

an antibacterial agent being a nanosilver;
a humectant being a glucan; and
a dispersant being a polymeric dispersant;
wherein a molecular weight of the dispersant is MWd, and the following condition is satisfied:

$$300000 \text{ g/mol} \leq MWd.$$

13. The contact lens composition of claim 12, wherein the molecular weight of the dispersant is MWd, and the following condition is satisfied:

$$500000 \text{ g/mol} \leq MWd.$$

14. The contact lens composition of claims 12 or 13, wherein a percentage of the nanosilver in the contact lens composition is Pag, a percentage of the dispersant in the contact lens composition is Pd, and the following condition is satisfied:

$$Pag/Pd \leq 0.0008.$$

15. The contact lens composition of claim 14, wherein the percentage of the nanosilver in the contact lens composition is Pag, and the following condition is satisfied:

$$0.00025\% \leq Pag.$$

16. The contact lens composition of claim 15, wherein the percentage of the dispersant in the contact lens composition is Pd, and the following condition is satisfied:

$$0.1\% \le Pd.$$

17. The contact lens composition of any one of claims 12 to 16, wherein a particle size of the nanosilver is Dag, and the following condition is satisfied:

$$Dag \le 200 \text{ nm.}$$

18. The contact lens composition of any one of claims 12 to 17, wherein the polymeric dispersant is a polyvinylpyrrolidone.

19. The contact lens composition of any one of claims 12 to 18, wherein a percentage of the humectant in the contact lens composition is Pm, and the following condition is satisfied:

$$0.5\% \le Pm \le 2.5\%.$$

20. The contact lens composition of any one of claims 12 to 19, wherein a K value of the dispersant is Kd, and the following condition is satisfied:

$$30 \le Kd.$$

21. The contact lens composition of any one of claims 12 to 20, wherein a particle size of the contact lens composition is Dc, and the following condition is satisfied:

$$Dc \le 500 \text{ nm.}$$

22. The contact lens composition of any one of claims 12 to 21, wherein an average transmittance in a wavelength range of 400 nm to 700 nm of the contact lens composition is T4070, and the following condition is satisfied:

$$85\% \le T4070.$$

23. The contact lens composition of claim 12, wherein a percentage of the nanosilver in the contact lens composition is Pag, a percentage of the dispersant in the contact lens composition is Pd, a percentage of the humectant in the contact lens composition is Pm, a particle size of the nanosilver is Dag, the molecular weight of the dispersant is MWd, a K value of the dispersant is Kd, an average transmittance in a wavelength range of 400 nm to 700 nm of the contact lens composition is T4070, and the following conditions are satisfied:

$$0.00042\% \le Pag \le 0.00048\%;$$

$$0.8\% \le Pd \le 1.5\%;$$

$$1.4\% \le Pm \le 1.6\%;$$

$$0.0004 \le Pag/Pd \le 0.0005;$$

$$70 \text{ nm} \le Dag \le 80 \text{ nm};$$

$$1200000 \text{ g/mol} \le MWd \le 1300000 \text{ g/mol};$$

$$85 \le Kd \le 95;$$

and

$$93\% \le T4070 \le 100\%.$$

**24.** A contact lens product, **characterized in** comprising:

the contact lens composition of any one of claims 12 to 23; and
a buffer solution, wherein the contact lens composition is immersed in the buffer solution.

Europäisches
Patentamt

European
Patent Office

Office européen
des brevets

# EUROPEAN SEARCH REPORT

**Application Number**

EP 25 19 2400

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 2 115 499 B1 (NOVARTIS AG [CH]) 11 November 2015 (2015-11-11) | 1,2,4-11 | INV. A01N59/16 |
| Y | * paragraphs [0054], [0056], [0078], | 12-24 | A01P1/00 |
| A | [0154]; claims 1-6 * | 3 | A61L12/08 |
| | ----- | | A01N25/30 |
| A | US 2013/224309 A1 (QIU YONGXING [US] ET AL) 29 August 2013 (2013-08-29) * paragraphs [0373] - [0375]; example 19 * | 1-24 | |
| | ----- | | |
| A | WO 2015/076840 A1 (AGIENIC INC [US]) 28 May 2015 (2015-05-28) * page 81; example 3 * | 1-24 | |
| | ----- | | |
| X | LINA M SHAKER ET AL: "Highly transparent antibacterial hydrogel-polymeric contact lenses doped with silver nanoparticles", JOURNAL OF VINYL AND ADDITIVE TECHNOLOGY, SOCIETY OF PLASTICS ENGINEERS, BROOKFIELD, CT, US, vol. 29, no. 6, 27 March 2023 (2023-03-27) , pages 1023-1035, XP072526967, ISSN: 1083-5601, DOI: 10.1002/VNL.21995 | 1,2,4-11 | |
| | | | TECHNICAL FIELDS SEARCHED (IPC) |
| Y | * paragraph [0004]; figure 3 * | 12-24 | A61L |
| A | | 3 | G02C |
| | ----- | | A01N |
| X | FERNANDO ISHARA ET AL: "Long term impact of surfactants & polymers on the colloidal stability, aggregation and dissolution of silver nanoparticles", ENVIRONMENTAL RESEARCH, ACADEMIC PRESS, SAN DIEGO, CA, US, vol. 179, 26 September 2019 (2019-09-26), XP085893293, ISSN: 0013-9351, DOI: 10.1016/J.ENVRES.2019.108781 [retrieved on 2019-09-26] | 1,2,4-11 | A01P |
| Y | * paragraphs [2.2.3], [03.3], [0004]; figures 2c, 3e, 5c * | 12-24 | |
| | ----- | | |
| | -/-- | | |

The present search report has been drawn up for all claims

| Place of search | Date of completion of the search | Examiner |
|---|---|---|
| Munich | 24 November 2025 | Sawicki, Marcin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................
& : member of the same patent family, corresponding document

EPO FORM 1503 03.82 (P04C01)

page 1 of 2

Europäisches
Patentamt

European
Patent Office

Office européen
des brevets

## EUROPEAN SEARCH REPORT

**Application Number**

EP 25 19 2400

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | RÓNAVÁRI ANDREA ET AL: "Polyvinyl-Pyrrolidone-Coated Silver Nanoparticles-The Colloidal, Chemical, and Biological Consequences of Steric Stabilization under Biorelevant Conditions", INTERNATIONAL JOURNAL OF MOLECULAR SCIENCES, vol. 22, no. 16, 12 August 2021 (2021-08-12), page 8673, XP093339233, Basel, CH ISSN: 1422-0067, DOI: 10.3390/ijms22168673 | 1,2,4-11 | |
| Y | * page 4, paragraph 3.1-3-3; figures 1-2 * | 12-24 | |
| Y | JP 2007 133001 A (SEED CO LTD; ADEKA CORP) 31 May 2007 (2007-05-31) * paragraph [0008]; claim 1 * | 12-24 | |
| Y,P | US 2025/170295 A1 (CHEN WEI-YUAN [TW] ET AL) 29 May 2025 (2025-05-29) * paragraphs [0041], [0043]; claims 1-28 * | 12-24 | **TECHNICAL FIELDS SEARCHED (IPC)** |

The present search report has been drawn up for all claims

| Place of search | Date of completion of the search | Examiner |
|---|---|---|
| Munich | 24 November 2025 | Sawicki, Marcin |

EPO FORM 1503 03.82 (P04C01)

## ANNEX TO THE EUROPEAN SEARCH REPORT
## ON EUROPEAN PATENT APPLICATION NO.

EP 25 19 2400

This annex lists the patent family members relating to the patent documents cited in the above-mentioned European search report.
The members are as contained in the European Patent Office EDP file on
The European Patent Office is in no way liable for these particulars which are merely given for the purpose of information.

24-11-2025

| Patent document cited in search report | | Publication date | Patent family member(s) | | Publication date |
|---|---|---|---|---|---|
| EP 2115499 | B1 | 11-11-2015 | CA | 2678598 A1 | 30-04-2009 |
| | | | EP | 2115499 A2 | 11-11-2009 |
| | | | HU | E027476 T2 | 28-09-2016 |
| | | | JP | 5675111 B2 | 25-02-2015 |
| | | | JP | 2011513767 A | 28-04-2011 |
| | | | KR | 20090115165 A | 04-11-2009 |
| | | | US | 2008203592 A1 | 28-08-2008 |
| | | | WO | 2009055082 A2 | 30-04-2009 |
| US 2013224309 | A1 | 29-08-2013 | AT | E442871 T1 | 15-10-2009 |
| | | | AU | 2004262891 A1 | 17-02-2005 |
| | | | BR | PI0411983 A | 29-08-2006 |
| | | | CA | 2530041 A1 | 17-02-2005 |
| | | | CN | 1822867 A | 23-08-2006 |
| | | | DK | 1648534 T3 | 07-12-2009 |
| | | | EP | 1648534 A1 | 26-04-2006 |
| | | | ES | 2333429 T3 | 22-02-2010 |
| | | | JP | 4624350 B2 | 02-02-2011 |
| | | | JP | 2009500645 A | 08-01-2009 |
| | | | MX | PA06000611 A | 11-04-2006 |
| | | | US | 2005013842 A1 | 20-01-2005 |
| | | | US | 2013224309 A1 | 29-08-2013 |
| | | | US | 2013228943 A1 | 05-09-2013 |
| | | | WO | 2005014074 A1 | 17-02-2005 |
| | | | ZA | 200510130 B | 27-12-2006 |
| WO 2015076840 | A1 | 28-05-2015 | EP | 3074024 A1 | 05-10-2016 |
| | | | WO | 2015076840 A1 | 28-05-2015 |
| JP 2007133001 | A | 31-05-2007 | NONE | | |
| US 2025170295 | A1 | 29-05-2025 | CN | 120065551 A | 30-05-2025 |
| | | | JP | 2025086876 A | 09-06-2025 |
| | | | TW | 202522091 A | 01-06-2025 |
| | | | US | 2025170295 A1 | 29-05-2025 |

EPO FORM P0459

For more details about this annex : see Official Journal of the European Patent Office, No. 12/82